(19) Europäisches Patentamt
European Patent Office
Office européen des brevets

(11) Veröffentlichungsnummer: **0 044 088**
**B1**

(12) # EUROPÄISCHE PATENTSCHRIFT

(45) Veröffentlichungstag der Patentschrift: **22.02.84**

(51) Int. Cl.³: **C 07 C 103/84,** C 07 D 207/38

(21) Anmeldenummer: **81106674.5**

(22) Anmeldetag: **09.02.79**

(60) Veröffentlichungsnummer der früheren Anmeldung nach Art. 76 EPÜ:

(54) **p-Methoxybenzoyl-Derivate.**

(30) Priorität: **10.02.78 CH 1403/78**
**22.11.78 CH 11981/78**

(43) Veröffentlichungstag der Anmeldung: **20.01.82 Patentblatt 82/3**

(45) Bekanntmachung des Hinweises auf die Patenterteilung: **22.02.84 Patentblatt 84/8**

(84) Benannte Vertragsstaaten: **BE CH DE FR GB IT LU NL SE**

(56) Entgegenhaltungen:
**FR - A - 2 100 836**

**CHEMICAL ABSTRACTS, Band 87, Nr. 5, 1. Augsut 1977, Seite 500, Nr. 39272m Columbus, Ohio, U.S.A.**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Kyburz, Emilio, Dr., Unterer Rebbergweg 127, CH-4153 Reinach (CH)**
Erfinder: **Aschwanden, Werner, Grellingerstrasse 4, CH-4107 Ettingen (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 044 088 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

p-Methoxybenzoyl-Derivate

Die vorliegende Erfindung betrifft p-Methoxybenzoyl-Derivate der allgemeinen Formel

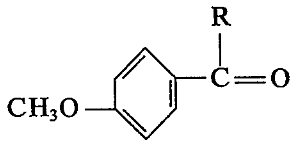

(I)

worin R einen Rest der Formel

$$-NH-(CH_2)_3-COOH$$ (a)

oder

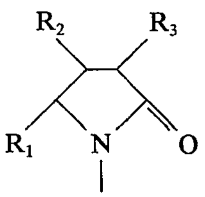

(b)

und eines von $R_1$ und $R_3$ Wasserstoff und das andere zusammen mit $R_2$ eine zweite C–C-Bindung bedeuten.

Die Verbindungen der Formel I eignen sich als Zwischenprodukte für die Herstellung von 1-(p-Methoxybenzoyl)-2-pyrrolidinon, welches die Formel

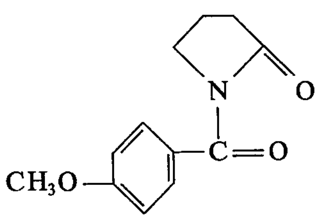

(II)

hat und welches eine neue Verbindung mit wertvollen pharmakodynamischen Eigenschaften ist, vgl. die Stammanmeldung No. 79.100379.1, jetzt Patent No. 0 005 143.

In FR-A-2 100 836 sind u. a. die 4-(3,4,5-Trimethoxybenzoylamino)-buttersäure und Salze davon als Wirkstoffe zur Behandlung oder Prophylaxe von Herzkrankheiten beschrieben. In ES-A-433 723, vgl. Chemical Abstracts 87 (1977), 39 272 m, ist beschrieben, daß das Cyclisationsprodukt der 4-(3,4,5-Trimethoxybenzoylamino)-buttersäure, das 1-(3,4,5-Trimethoxybenzoyl)-2-pyrrolidinon Lern- und Erinnerungsprozesse bei Ratten erleichtert.

Die Verbindung der Formel I, worin R den Rest der Formel (a) bedeutet, d. h. die 4-(p-Methoxybenzoylamino)-buttersäure, kann dadurch hergestellt werden, daß man 4-Aminobuttersäure entsprechend acyliert, beispielsweise mittels p-Methoxybenzoylchlorid in Gegenwart eines säurebindenden Mittels, wie Natronlauge.

Verbindungen der Formel I, worin R einen Rest der Formel (b) bedeutet können dadurch hergestellt werden, daß man 5-Oxo-3-pyrrolidinylacetat oder einen anderen geeigneten Ester des 5-Oxo-3-pyrrolidinols mit Trimethylchlorsilan o. dgl. behandelt, die entstandene Verbindung, z. B. das 5-Oxo-1-trimethylsilyl-3-pyrrolidinylacetat, mit einem geeigneten p-Methoxybenzoylierungsmittel (wie p-Methoxybenzoylchlorid) umsetzt und das erhaltene Produkt unter milden Bedingungen mit einer Base (wie Natriumbicarbonat) behandelt oder daß man eine Verbindung der allgemeinen Formel

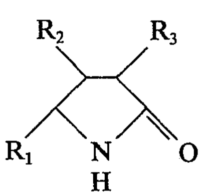

(III)

worin $R_1$, $R_2$ und $R_3$ die eingangs erwähnte Bedeutung besitzen,
bzw. ein Gemisch der beiden unter die allgemeine Formel III fallenden Verbindungen in 1-Stellung

entsprechend acyliert.

Das 1-(p-Methoxybenzoyl)-2-pyrrolidinon der Formel II kann aus den Verbindungen der Formel I dadurch hergestellt werden, daß man

a) eine Verbindung der Formel I, worin R einen Rest der Formel (b) bedeutet, d. h. ein 2-Pyrrolidinon-Derivat der allgemeinen Formel

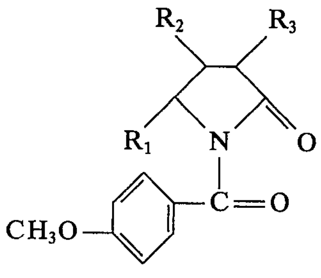

(Ia)

worin $R_1$, $R_2$ und $R_3$ die eingangs erwähnte Bedeutung besitzen, bzw. ein Gemisch der beiden unter die allgemeine Formel Ia fallenden Verbindungen reduziert oder

b) die Verbindung der Formel I, worin R den Rest der Formel (a) bedeutet, d. h. die 4-(p-Methoxybenzoylamino)buttersäure, cyclisiert.

Für die Reduktion einer Verbindung der Formel Ia bzw. eines Gemisches der beiden unter die Formel Ia fallenden Verbindungen verwendet man Methoden, welche für derartige Reduktionen an sich allgemein gebräuchlich sind. Vorzugsweise erfolgt diese Reduktion mittels katalytisch erregtem Wasserstoff in einem unter den Reaktionsbedingungen inerten organischen Lösungsmittel, wobei als Hydrierungskatalysatoren Palladium, Platin und dergleichen in Frage kommen und als Lösungsmittel beispielsweise Essigester, Alkohole (wie Methanol, Äthanol oder dergleichen), Äther (wie Tetrahydrofuran) usw.

Bei der Cyclisation der 4-(p-Methoxybenzoylamino)-buttersäure wird 1 Mol Wasser abgespalten, und man verwendet demnach hierfür Methoden, welche für derartige dehydratisierende Cyclisationen an sich allgemein gebräuchlich sind, d. h. Erhitzen und/oder Behandlung mit einem Wasserabspaltenden Mittel, wie Polyphosphorsäure, Phosphorpentachlorid, Thionylchlorid und dergleichen. Je nach der für die dehydratisierende Cyclisation angewendeten Methode kann es von Vorteil sein, in einem unter den Reaktionsbedingungen inerten organischen Lösungsmittel zu arbeiten, beispielsweise in einem aromatischen Kohlenwasserstoff, wie Toluol o. dgl., in einem Äther, wie Tetrahydrofuran oder dergleichen, in einem halogenierten Kohlenwasserstoff, wie Chloroform oder dergleichen usw.

Wie eingangs erwähnt, ist das 1-(p-Methoxybenzoyl)-2-pyrrolidinon der Formel II eine neue Verbindung mit wertvollen pharmakodynamischen Eigenschaften. Die Verbindung der Formel II weist nur eine sehr geringe Toxizität auf, und es hat sich gezeigt, daß sie in den nachstehend beschriebenen Tierversuchen experimentell auf verschiedene Weise hervorgerufener cerebraler Insuffizienz entgegenzuwirken vermag.

### A) Posthypercapnische »Avoidance«-Acquisition

Die Test-Apparatur ist eine »shuttle box« mit einer 10 cm hohen Hürde in der Mitte und elektrifizierbarem Gitterboden. In der schalldichten Kammer ist ein Lautsprecher montiert. Eine oder drei Stunden nach Verabreichung von Kontroll- oder Präparat-Injektion werden unerfahrene Ratten (120 – 150 g; 10 pro Gruppe) für 12 Sekunden in reines $CO_2$-Milieu gebracht. Eine dritte Gruppe von 10 Ratten wird weder mit dem Präparat noch mit $CO_2$ behandelt. Drei Minuten nach $CO_2$-Behandlung müssen die Ratten aller drei Gruppen in der »shuttle box« in folgendem Programm einen bedingten und unbedingten Reflex erlernen: 10 Sek. Stille – 5 Sek. Ton (»avoidance response«) – 15 Sek. Ton + Fuß-Schock (»escape response«); sechsmal hintereinander. Für jeden der sechs Einzelversuche wird die Reaktionszeit (Zeit bis die Ratte über die Hürde springt) jeder Ratte gemessen und die statistische Signifikanz der Unterschiede zwischen den verschiedenen Gruppen mittels Rang-Test berechnet.

Als »aktiv« bezeichnet man diejenige Dosis eines Präparats, welche während der sechs Einzelversuche eine signifikante Wirkung zeigt; dabei müssen die mit dem Präparat und $CO_2$ behandelten Tiere signifikant besser lernen als die nur mit $CO_2$ behandelten Tiere und gleich gut wie die weder mit dem Präparat noch mit $CO_2$ behandelten Tiere.

B) »Passive Avoidance«-Test mit Elektroschock-Amnesie

Die Testapparatur ist eine Skinnerbox mit elektrifizierbarem Gitterboden und einer grauen Viereckplattform in einer Ecke. Unerfahrene männliche Ratten, 100–120 g schwer, werden auf die graue Plattform gesetzt. Jedesmal wenn sie auf den Gitterboden hinuntersteigen, erhalten sie einen Elektroschock. Nach 3–5 Versuchen zeigen die Ratten eine sogenannte »passive avoidance response« d. h. Weigerung von der Plattform herunterzusteigen. Unmittelbar nach Acquisition der Weigerung werden drei Gruppen zu je 20 Ratten gebildet. Eine Gruppe erhält einen Elektroschock (45 mA, 2 Sek.) durch die Ohren und eine i. p. NaCl-Injektion. Die zweite Gruppe erhält einen Elektroschock durch die Ohren und eine i. p. Injektion der Testsubstanz. Die dritte Gruppe erhält nur NaCl. Nach drei Stunden wird jede Ratte einmal auf die Plattform gesetzt und die Verweildauer (max. 60 Sek.) gemessen. Die signifikante Wirkung der Testsubstanz im Vergleich zu den beiden Kontrollgruppen wird mittels Rang-Test berechnet. Als »aktiv« bezeichnet man diejenige Dosis eines Präparates, welche eine signifikante Schutzwirkung gegen den Elektroschock zeigt (die mit aktiver Dosis eines Präparats und Elektroschock behandelten Tiere zeigen eine lange Verweildauer, ebenso die nicht mit Elektroschock behandelten Tiere, wogegen die mit NaCl und Elektroschock behandelten Tiere eine kurze Verweildauer zeigen).

C) Verzögerung des Haloperidol-induzierten »knock out« in einem »Continuous Avoidance«-Programm mit Totenkopfaffen

Männliche, erwachsene Totenkopfaffen (Saimiri sciureus), 0,6 bis 1,2 kg schwer, einzeln gehalten, werden in einer 2-Tasten Skinnerbox auf folgendes »continuous avoidance«-Programm trainiert: »Avoidanceshock«-Intervall 40 Sek.; »shock-shock«-Intervall 20 Sek.; Fuß-Schock max. 5 Sek. Affen mit regelmäßiger Grundleistung erhalten Haloperidol 1,0 mg/kg p. o. zur Bestimmung der »knock-out«-Zeit. (Blockierung von »avoidance« und »escape«). Affen mit stabilen »knock-out«-Zeiten werden für die Evaluation von Versuchspräparaten als potentielle cerebrale Insuffizienzverbesserer selektioniert. Die Präparate können zu verschiedenen Zeiten vor der Behandlung mit Haloperidol injiziert werden. Als »aktiv« bezeichnet man diejenige Dosis eines Präparates, welche bei Verabreichung vor der Behandlung mit Haloperidol eine signifikante Verzögerung des »knock out«Zeitpunkts in einem drei Stunden-Test bewirkt.

D) Anti-Anoxie-Test

Männliche Ratten im Gewicht von 300–350 g werden für die Versuche verwendet. Unter Halothannarkose werden die Tiere tracheotromiert und je eine epidurale Cortexelektrode gesetzt. Nach Abschluß der Präparation wird die Narkose abgesetzt, sämltiche Wunden und Druckstellen werden mit Xylocain infiltriert, d-Tubocurarin wird infundiert, und das Tier wird künstlich beatmet. Das EEG wird während der ganzen Versuchsdauer kontinuierlich aufgezeichnet. Anoxien werden in stündlichen Abständen durchgeführt, indem das Tier mit 99,9% $N_2$ beatmet wird bis zum Erreichen eines isoelektrischen EEG. Nach einer Periode von 30 Sekunden mit isoelektrischem EEG wird wieder mit normaler Raumluft beatmet.

Als Prüfgrößen werden definiert:

1. »survival time«:
   = ST — Zeit bis zum Erreichen eines isoelektrischen EEG unter $N_2$-Beatmung.
2. »recovery time«:
   = RT — Zeit zwischen Wiederbeatmung mit Raumluft und erster elektrischer Aktivität im Cortex.

Die Substanzen werden 60 oder 120 Minuten vor der ersten Anoxie verabreicht. Die ST- und RT-Werte von behandelten Ratten werden mittels Rang-Test mit Placebobehandelten Kontrollen verglichen. Eine Verlängerung von ST und/oder Verkürzung von RT wird als Schutzwirkung gegen eine Anoxie angesehen. Als »aktiv« wird diejenige Dosis eines Präparates bezeichnet, welche eine signifikante derartige Schutzwirkung entfaltet.

In den vorstehend beschriebenen Versuchen zeigt das 1-(p-Methoxybenzoyl)-2-pyrrolidinon, welches eine sehr geringe akute Toxizität aufweist [DL 50 >5000 mg/kg p. o. (Maus)] bereits bei den folgenden Dosen eine signifikante Aktivität:

| Versuch | Erste signifikant wirksame Dosis |
|---|---|
| A | 10 mg/kg i.p. (nach 1 Std.) |
| | 30 mg/kg p.o. (nach 1 Std.) |
| B | 50 mg/kg i.p. |
| C | 1 mg/kg i.p. |
| | 0,1 mg/kg p.o. |
| D | 20 mg/kg i.p. |

Das 1-(p-Methoxybenzoyl)-2-pyrrolidionon der Formel II kann als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von Tabletten, Lacktabeltten, Dragées und Hartgelatinekapseln kann das 1-(p-Methoxybenzoyl)-2-pyrrolidinon mit pharmazeutisch inerten, anorganischen oder organischen Excipientien verarbeitet werden. Als solche Excipientien kann man z. B. für Tabletten, Dragées und Hartgelatinekapseln Lactose, Maisstärke oder Derivate davon, Talk, Stearinsäure oder deren Salze etc. verwenden.

Für Weichgelatinekapseln eignen sich als Excipientien z. B. vegetabile Öle, Wachse, Fette, halbfeste und flüssige Polyole etc.

Zur Herstellung von Lösungen und Sirupen eignen sich als Excipientien z. B. Wasser, Polyole, Saccharose, Invertzucker, Glukose und dergleichen.

Für Injektionslösungen eignen sich als Excipientien z. B. Wasser, Alkohole, Polyole, Glyzerin, vegetabile Öle etc.

Für Suppositorien eignen sich als Excipientien z. B. natürliche oder gehärtete Öle, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Das 1-(p-Methoxybenzoyl)-2-pyrrolidinon der Formel II kann man bei der Bekämpfung bzw. Verhütung der cerebralen Insuffizienz bzw. bei der Verbesserung der intellektuellen Leistungsfähigkeit verwenden, beispielsweise bei cerebralen Insulten, in der Geriatrie, bei Alkoholismus usw. Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 10 bis 2500 mg 1-(p-Methoxybenzoyl)-2-pyrrolidinon angemessen sein, wobei aber die soeben angegebene obere Grenze im Hinblick auf die geringe Toxizität des 1-(p-Methoxybenzoyl)-2-pyrrolidinons ohne weiteres überschritten werden kann, wenn sich dies als angezeigt erweisen sollte.

In den nachfolgenden Beispielen, welche die vorliegende Erfindung illustrieren, ihren Umfang jedoch in keiner Weise einschränken sollen, sind sämtliche Temperaturen in Celsiusgraden angegeben.

## Beispiel 1

10,0 g 5-Oxo-3-pyrrolidinylacetat und 8,82 ml Trimethylchlorsilan werden in 100 ml abs. Tetrahydrofuran gelöst, worauf bei −5° bis 0° 9,65 ml Triäthylamin zugegeben werden. Man beläßt die Temperatur 1 Stunde bei −5° bis 0°, worauf man unter Argonatmosphäre abfiltriert, das Filtrat eindampft und den Rückstand im Vakuum destilliert. Man erhält 5-Oxo-1-trimethylsilyl-3-pyrrolidinylacetat vom Siedepunkt 110°/0,07 mmHg.

12,5 g 5-Oxo-1-trimethylsilyl-3-pyrrolidinylacetat werden in 30 ml abs. Tetrahydrofuran vorgelegt, worauf bei 0–10° 9,90 g p-Methoxybenzoylchlorid, gelöst in 10 ml abs. Tetrahydrofuran, zugetropft werden. Man rührt 1 Stunde bei Raumtemperatur und 1 Stunde bei 70° und dampft danach ein. Der Rückstand wird in Essigester gelöst, und die Essigesterlösung wird mit Natriumchlorid- und

Natriumbicarbonatlösung gewaschen, über Natriumsulfat getrocknet und eingedampft. Zum zurückbleibenden viskosen Öl werden 20 ml Tetrahydrofuran und 20 ml gesättigte Natriumbicarbonatlösung gegeben, worauf bei Raumtemperatur 18 Stunden gerührt wird. Das Lösungsmittelgemisch wird abdekantiert, und der Rückstand wird zwischen Wasser und Methylenchlorid verteilt. Die organische Phase wird abgetrennt, über Natriumsulfat getrocknet und eingedampft. Das zurückbleibende braune Öl wird über Kieselgel (Korngröße 0,2 – 0,5 mm) chromatographiert. Das mit Benzol-Diäthyläther (Gemisch 1 : 1) eluierte 1-(p-Methoxybenzoyl)pyrrolin-2-on wird aus Alkohol umkristallisiert und zeigt dann einen Schmelzpunkt von 148 – 150°.

Das 1-(p-Methoxybenzoyl)pyrrolin-2-on kann wie folgt in 1-(p-Methoxybenzoyl)-2-pyrrolidinon übergeführt werden:

150 mg 1-(p-Methoxybenzoyl)pyrrolin-2-on wird in 100 ml Essigsäureäthylester gelöst und über 30 mg 5%iger Palladiumkohle mit Wasserstoff bei Atmosphärendruck und Raumtemperatur hydriert. Nach Abfiltrieren des Katalysators und Abdampfen des Lösungsmittels erhält man 1-(p-Methoxybenzoyl)-2-pyrrolidinon.

## Beispiel 2

Zu 30,9 g 4-Aminobuttersäure, 24,0 g Natriumhydroxid und 300 ml ionenfreiem Wasser werden bei 30° unter gutem Rühren 10,2 g p-Methoxybenzoylchlorid in einer Portion zugegeben. Nach 2 Stunden wird bei einer Temperatur unterhalb von 10° mit 75 ml konz. Salzsäure angesäuert. Der Niederschlag wird abfiltriert, mit Wasser ionenfrei gewaschen, im Trockenschrank bei 65° über Phosphorpentoxid getrocknet und dann aus 45 ml eines Gemisches von Aceton und tiefsiedendem Petroläther (Verhältnis 3 : 1) umkristallisiert. Man erhält 4-(p-Methoxybenzoylamino)buttersäure vom Schmelzpunkt 120,5 – 122°.

Die 4-(p-Methoxybenzoylamino)buttersäure kann wie folgt in 1-(p-Methoxybenzoyl)-2-pyrrolidinon übergeführt werden:

a) 10 g Phosphorpentoxid und 6 ml ortho-Phosphorsäure (mindest 85%ig) werden miteinander erwärmt. Zu der entstehenden Lösung werden bei Raumtemperatur 2,0 g 4-(p-Methoxybenzoylamino)buttersäure zugegeben. Das Reaktionsgemisch wird 60 Minuten auf 50° erwärmt, anschließend mit Eis versetzt und mit Essigester extrahiert. Die organische Phase wird zunächst mit kaltem Wasser, dann mit kalter Natriumbicarbonatlösung und schließlich nochmals mit Wasser gewaschen und über Natriumsulfat getrocknet. Der Rückstand wird mit Diäthyläther verrührt, und man erhält 1-(p-Methoxybenzoyl)-2-pyrrolidinon vom Schmelzpunkt 120 – 122°.

b) 20,0 g 4-(p-Methoxybenzoylamino)buttersäure, 50 ml Toluol und 9,2 ml Thionylchlorid werden 2 Stunden am Rückfluß erwärmt, worauf man mit Entfärbungskohle behandelt und eindampft. Der Rückstand wird in Methylenchlorid gelöst und über neutralem Aluminiumoxid chromatographiert. Das mit Methylenchlorid eluierte 1-(p-Methoxybenzoyl)-2-pyrrolidinon wird aus Alkohol umkristallisiert und zeigt einen Schmelzpunkt von 119 – 120°.

**Patentansprüche**

1. p-Methoxybenzoyl-Derivate der allgemeinen Formel

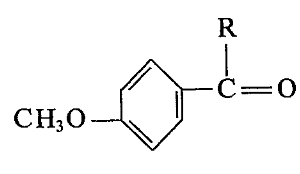

(I)

worin R einen Rest der Formel

$$—NH—(CH_2)_3—COOH \qquad (a)$$

oder

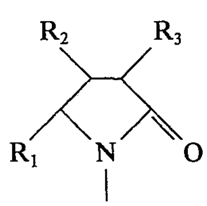

(b)

und eines von $R_1$ und $R_3$ Wasserstoff und das andere zusammen mit $R_2$ eine zweite C–C-Bindung bedeuten.

2. 4-(p-Methoxybenzoylamino)buttersäure.

## Claims

1. p-Methoxybenzoyl derivatives of the general formula

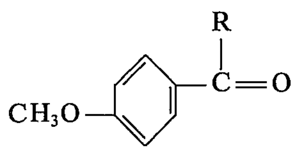

(I)

wherein R signifies a residue of the formula

$$—NH—(CH_2)_3—COOH \quad (a)$$

or

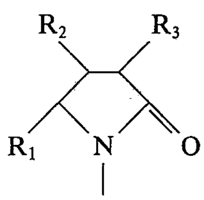

(b)

and one of $R_1$ and $R_3$ signifies hydrogen and the other together with $R_2$ signifies a second C–C-bond.

2. 4-(p-Methoxybenzoylamino)butric acid.

## Revendications

1. Dérivés de p-méthoxybenzoyle de formule générale

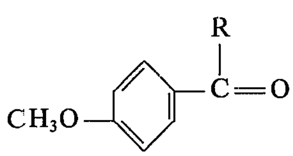

(I)

où R représente un radical de formule

$$—NH—(CH_2)_3—COOH \quad (a)$$

ou

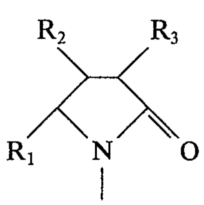

(b)

et l'un des radicaux $R_1$ et $R_3$ représente un hydrogène et l'autre forme avec $R_2$ une deuxième liaison C–C.

2. Acide 4-(p-méthoxybenzoylamino)butyrique.